(19) Europäisches Patentamt

European Patent Office

Office européen des brevets

(11) Publication number: **0 253 619 B1**

(12) **EUROPEAN PATENT SPECIFICATION**

(45) Date of publication of patent specification: **15.01.92**   (51) Int. Cl.⁵: **A61K 9/50**

(21) Application number: **87306202.0**

(22) Date of filing: **14.07.87**

(54) **Method of preparing single bilayered liposomes.**

(30) Priority: **15.07.86 US 885619**

(43) Date of publication of application:
**20.01.88 Bulletin 88/03**

(45) Publication of the grant of the patent:
**15.01.92 Bulletin 92/03**

(84) Designated Contracting States:
**AT BE CH DE ES FR GB GR IT LI LU NL SE**

(56) References cited:
**EP-A- 0 107 559**
**EP-A- 0 140 085**
**EP-A- 0 190 926**

(73) Proprietor: **CILAG LTD.**
**Hochstrasse 201/209**
**CH-8201 Schaffhausen(CH)**

(72) Inventor: **Boller, Fritz H.**
**Punten Strasse 27**
**CH-8185 Winkel(CH)**
Inventor: **Niederer, Roland R.**
**IM Landgut**
**CH-8211 Stetten(CH)**

(74) Representative: **Jones, Alan John et al**
**CARPMAELS & RANSFORD 43 Bloomsbury**
**Square**
**London, WC1A 2RA(GB)**

Rank Xerox (UK) Business Services

**Description**

The present invention relates to a method of preparing single bilayered liposomes. More specifically, the present invention relates to an improved method for producing single bilayered liposomes which contain encapsulated biologically active materials.

Liposomes are widely described in the literature and their structure is well known. They are formed by amphiphathic molecules such as polar lipids, examples of which include phosphatidyl cholines, ethanolamines and serines, sphingomyelins, cardiolipins, plasmalogens, phosphatidic acids and cerebrosides. Liposomes are formed when phospholipids or other suitable amphiphathic molecules are allowed to swell in water or aqueous solutions to form liquid crystals usually of multilayer structure comprised of many bilayers separated from each other by aqueous material. Another type of liposome is known consisting of a single bilayer encapsulating aqueous material which may also be referred to as a unilamellar vesicle. If water-soluble materials are included in the aqueous phase during the swelling of the lipids they become entrapped between the lipid bilayers.

In recent years there has been much interest in the use of liposomes as carriers of compounds which are of interest because of some biological property, for example, medicaments, proteins, enzymes, hormones and diagnostic agents, hereinafter referred to as "biologically active compounds".

Water-soluble materials are encapsulated in the aqueous spaces between the biomolecular layers. Lipid soluble materials are incorporated into the lipid layers although polar head groups may protrude from the layer into the aqueous space. The encapsulation of these compounds can be achieved by a number of methods. The method most commonly used involves casting a thin film of phospholipid onto the walls of a flask by evaporation of an organic solvent. When this film is dispersed in a suitable aqueous medium, multilamellar liposomes are formed (also referred to as coarse liposomes). Upon suitable sonication, the coarse liposomes form smaller similarly closed vesicles.

Water-soluble biologically active materials are usually incorporated by dispersing the cast film with an aqueous solution of the compound. The unencapsulated compound is then removed by centrifugation, chromatography, dialysation or some other suitable procedure. Lipid-soluble biologically active materials are usually incorporated by dissolving them in the organic solvent with the phospholipid prior to casting the film. If the solubility of these materials in the lipid phase is not exceeded or the amount present is not in excess of that which can be bound to the lipid, liposomes prepared by the above method usually contain most of the material bound in the lipid bilayers; separation of the liposomes from unencapsulated material is not required.

Liposomes are lipid vesicles comprising one or more lipid layers dispersed in water or an aqueous electrolyte solution. Although they are widely used to encapsulate biologically active materials for a variety of purposes, liposomes are generally used as drug carriers.

Various types of liposomes have been utilized, depending upon the number of lipid layers, size, surface charge, lipid composition and method of preparation.

A method for preparing multilamellar lipid vesicles is described by Bangham et al. [(J. Mol. Biol. 13:238-252 (1965)] Multilamellar lipid vesicles are composed of a number of bimolecular lamellae interspersed with an aqueous medium. The lipids and lypophilic substances are first dissolved in an organic solvent. The solvent is removed under reduced pressure by rotary evaporation and the lipid residue forms a thin film on the wall of the container. Upon the addition of an aqueous solution, generally containing electrolytes and/or hydrophilic biologically active materials, large multilamellar liposomes are formed when the mixture is agitated. Small unilamellar vesicles can be prepared by sonication of the large multilamellar vesicles.

Alternatively, a mixture of the lipid and an aqueous solution is warmed and then subjected to vigorous agitation and ultrasonic vibration. O. Zumbuehl and H. G. Weder describe an approach in which the lipids and additives are solubilized with detergents by agitation or sonication, yielding defined mixed micelles. The detergents are then removed by dialysis [(Biochem. Biophys. Acta. 640:252-262, (1981)]. A more recent method for preparing large unilamellar lipid vesicles is the reverse phase evaporation techniques described in U. S. Patent No. 4,235,871. This technique consists of forming a water-in-oil emulsion. Removal of the organic solvent under reduced pressure results in a mixture having a gel-like character which can then be converted to lipid vesicles by agitation or by dispersion in an aqueous medium.

Several techniques for making unilamellar vesicles have been reported. For example, the sonication of an aqueous dispersion of phospholipid results in microvesicles consisting of bilayer or phospholipid surrounding an aqueous space [(Papahadjopoulos and Miller, Biochem. Biophys. Acta., 135:224-238, (1968)].

All of the above methods produce reproducible liposomes in laboratory batch size. Scaling up to

2

production batch size using known procedures is often difficult or not feasible at all depending upon the equipment employed due in part to the difficulty encountered in duplicating the physical conditions which give rise to liposome formation. One method of overcoming this problem involves the use of the ether infusion technique described by D. Deamer and A. D. Bangham [(Biochem. Biophys. Acta 443:629-634, (1967)]. In this process the ether solution containing the lipids is injected rapidly into a buffer solution at 60°C, whereupon it spontaneously forms liposomes of the unilamellar type as the ether evaporates. The injection method is simple and rapid but results in a relatively dilute preparation of liposomes and provides low encapsulation efficiency.

An alternate method for the preparation of small unilamellar vesicles that avoids the need of sonication is the ethanol injection technique. [(S. Batzri and E. D. Korn, Biochem. Biophys. Acta. 298:1015-1019, (1973)]. Single bilayered liposomes are prepared by injecting an ethanolic solution of phospholipid into water. The suspension is concentrated by ultrafiltration and the ethanol is removed by dialysis.

In U.S. Patent No. 4,206.197 a process for coating chemicals is described wherein a fat is mixed with a water soluble solvent, surfactant and the chemical to be coated. The solution is then pressure injected into the aqueous phase. The product formed by this process, however, is a colloidal suspension.

A comprehensive review of the types of liposomes and methods for preparing them is contained in "Liposome Technology", Ed. by G. Gregoriadis, CRC Press Inc.. Boca Raton, Florida, Vols. I, II & III (1984).

As indicated above, the known techniques for preparing single bilayered liposomes produce liposomes in laboratory batch size but often cannot be adapted to production scale at all or can be adapted only with difficulty. It is an object of the present invention to provide a process for preparing single bilayered liposomes on a large scale. It is another object of this invention to provide a method of encapsulating biologically active materials on a large scale.

The present invention provides a process for encapsulating biologically active materials on a large scale in single bilayered liposomes which comprises dissolving a lipid component in a suitable organic solvent, injecting the organic component directly into an aqueous component under pressure and simultaneously mixing the organic and aqueous phases with a high speed mixing means, whereupon the liposomes are formed spontaneously.

The single bilayered liposomes containing encapsulated biologically active material can be employed directly or they can be employed in a suitable pharmaceutically acceptable carrier for topical or systemic administration. The viscosity of the liposomes can be increased by the addition of one or more suitable thickening agents such as xanthan gum, hydroxypropyl cellulose, hydroxypropyl methylcellulose or a mixture thereof.

Because no further treatment of the liposomes such as ultrasonificaticn, filtration, centrifugation or dialysis, is required prior to use, the process of this invention can be used for encapsulating biologically active materials on a large scale suitable for use in the pharmaceutical industry.

In accordance with the present invention, a process is provided for preparing single bilayered liposomes. The process can be employed to make liposomes in large scale production batches.

By the process of the present invention, single bilayered liposomes containing encapsulated biologically active material are obtained by injecting under pressure, an organic solution of the biologically active material into an aqueous component while simultaneously mixing the organic and aqueous components with a high speed homogenizer or mixing means.

The aqueous component consists of water alone or it may contain electrolytes, buffered systems and other ingredients, such as preservatives. Suitable electrolytes which can be employed include metal salts such as the alkali metal and alkaline earth metal salts. The preferred metal salts are calcium chloride, sodium chloride and potassium chloride. The concentration of the electrolyte may vary over a wide range from zero to 260 mM. The preferred range is 5 mM to 160 mM. The aqueous component is placed in a suitable vessel which can be adapted to effect homogenization by effecting great turbulence during the injection of the organic component. Homogenization of the two components can be accomplished within the vessel, or, alternatively, the aqueous and organic components may be injected separately into a mixing means which is located outside the vessel. In the latter case, the liposomes are formed in the mixing means and then transferred to another vessel for collection purposes.

The organic component consists of a suitable non-toxic, pharmaceutically acceptable solvent such as ethanol, glycerol, propylene glycol or polyethylene glycol, and a suitable phospholipid which is soluble in the solvent. Suitable phospholipids which can be employed include lecithin, phosphatidylcholine, phosphatidylethanolamine, phosphatydylserine, phosphatidylinositol, lysophosphatidylcholine and phosphatidyl glycerol. Other lipophilic additives may be employed in order to modify selectively the characteristics of the liposomes. Examples of such other additives include stearylamine, phosphatidic acid, tocopherol, cholesterol and lanolin extracts.

The biologically active material is dissolved in the organic component. Any biologically active material which is soluble in the organic solvent may be employed. As used in the specification and claims, the term biologically active material means a compound or composition which, when present in an effective amount, produces an effect in living cells or organisms. Examples of biologically active materials useful in this invention include but are not limited to dermatological agents, (e.g. triamcinolone acetonide, retinoic acid, 13-cis retinoic acid, hydrocortisone): anti-bacterial agents (e.g. ampicillin); antifungal agents (e.g. econazole base, econazole nitrate, miconazole, butaconazole, terconazole, amphotericine B): antisecretroy agents (e.g. cimetidine, ranitidine); anti-convulsants (e.g. diphenylhydantoin): anti-hypertensive agents (e.g. minoxidil); anti-cancer agents (e.g. methotrexate); immunomodulators (e.g. lipophillic derivatives of muramyl dipeptide); anti-viral agents (e.g. acyclovir, interferons); non-steroidal anti-inflammatory agents (e.g. ibuprofen, suprofen); and prostaglandins (e.g. rioprostil, mesoprostil).

It may be advantageous to use micronized forms of the biologically active material, i.e. material having an average particle size of less than 10 $\mu$m, as the high surface area will facilitate the dissolution of the liposomal components.

In addition, other ingredients which can prevent oxidation of the phospholipids may be added to the organic component. Examples of such other ingredients include tocopherol, butylated hydroxyanisole, butylated hydroxytoluene, ascorbyl palmitate and ascorbyl oleate. Preservatives such as benzoic acid, methyl paraben and propyl paraben may also be added.

The volume of the organic component will vary depending upon the solubility of the ingredients. In general, sufficient solvent must be employed to dissolve all lipophilic ingredients but the solvent should not exceed 10% of the entire formulation.

Any high speed mixing means or homogenizer which will allow for high speed mixing of the components under pressure may be employed. Generally a mixer capable of speeds from 1500 to 20,000 rpm is employed. In a preferred method, the organic component is injected into the aqueous component through one or more nozzles attached to the mixing means. It is preferred to use an homogenizer having a nozzle diameter of 0.1 to 20 mm, but any suitable nozzle diameter may be employed. The preferred diameter is 0.1 to 10 mm. The pump employed with the homogenizer should be capable of effecting a pressure of 0.1 to 300 bar (0.01 to 21.4 psi). The flow rate of the organic component during injection can vary between 1 ml/min and 1000 ml/min. Independent of the batch size, the flow rate of the aqueous solution should exceed at least 50 times the flow rate of the organic solution, when the two components are mixed in a separate vessel. The actual flow rate employed will depend upon the speed capability of the particular mixing means employed. It is preferred to purge the solutions with an inert gas such as nitrogen or argon to prevent possible oxidation of the materials employed during the procedure. The liposomes produced by the process of this invention are collected by techniques known to those skilled in the art. Liposomes have been found to be most stable at a pH between 5-7. Any suitable mineral or organic acid such as hydrochloric acid, hydrobromic acid or acetic acid may be added to the liposomes until the desired pH range is attained.

The liposomes can be used, for example, as carriers for biologically and/or pharmacodynamically active substances and/or themselves constitute pharmaceutical preparations. The liposome formulations prepared as described above can be administered topically or orally as the case may be. They can be administered topically without further purification. In the case of oral administration, further purification of the liposome may be necessary to remove potentially toxic materials. Depending upon the particular mode of administration, it may be desirable to administer the liposome in a suitable pharmaceutically acceptable carrier. Examples of such carriers include jellies, solutions and aerosols.

The present method is useful for preparing liposomes that include a biologically active material. However, it is apparent from the present teachings that the single bilayered liposomes can be prepared minus the active material. Preferred embodiments of the present invention are now described by way of example only.

## EXAMPLE I

| Composition | |
| --- | --- |
| Econazole base (micronized) | 2.5 g |
| Phosphatidylcholine | 25.0 g |
| Cholesterol | 2.5 g |
| Benzoic acid | 0.5 g |
| Butylated hydroxyanisole | 0.0125 g |
| Ethanol | 20.0 g |
| Calcium chloride solution (8mM) | 199.488 g |

Procedure

Econazole base, phosphatidylcholine, cholesterol, benzoic acid and butylated hydroxyanisole were added to the ethanol and the mixture was heated at 45-50°C until the materials dissolved. The calcium chloride solution was placed in a high performance homogenizer [POLYTRON (PTA2SM) manufactured by Kinematica Littau, Lucerne, Switzerland] at 25°C. The ethanol solution was then pumped through a tube directly into the calcium chloride solution and the solutions were simultaneously mixed at high speed. Liposomes having a diameter less than 2.5 $\mu$m were spontaneously formed.

Technical Data

Homogenizer speed 20,000 rpm
Flow rate 8 ml/min.
Diameter of the nozzle opening 0.18 mm
Pressure 100-150 bar (7-10 psi)

EXAMPLE II

Composition

Econazole base (micronized) 2.5 g
Phosphatidylcholine 25.0 g
Cholesterol 2.5 g
Methylparaben 0.35 g
Propylparaben 0.025 g
Butylated hydroxytoluene 0.025 g
Ethanol 12.5 g
Calcium chloride solution (8mM) 207.1 g

Procedure

Methylparaben, propylparaben, butylated hydroxytoluene, cholesterol, phosphatidylcholine, and econazole base were added to the ethanol and the suspension was heated at 45-50°C until the materials dissolved. The suspension was purged with nitrogen during the dissolution process. The calcium chloride solution was placed in a high performance homogenizer [POLYTRON (PTA2SM) manufactured by Kinematica Littau, Lucerne, Switzerland] at 20°C. The ethanol solution was then pumped through a tube directly into the calcium chloride solution and the solutions were simultaneously mixed at high speed. Each solution was purged with nitrogen during the mixing procedure. Liposomes having a diameter less than 3 $\mu$m were spontaneously formed.

Technical Data

Homogenizer speed 9000 rpm
Flow rate 8 ml/min.
Diameter of the nozzle opening 1 mm
Pressure 30 bar (2 psi)

EXAMPLE III

Batch size 18 kg

| Composition | |
|---|---|
| Econazole base (micronized) | 180.0 g |
| Lecithin | 1800.0 g |
| Cholesterol | 180.0 g |
| Methylparaben | 25.2 g |
| Propylparaben | 1.8 g |
| Sodium chloride | 19.98 g |
| Water purified | 13480.02 g |
| Ethanol | 1800.0 g |
| Ascorbylpalmitate | 18.0 g |
| Hydroxypropylmethyl cellulose | 396.0 g |
| Perfume | 36.0 g |
| Hydrochloric acid 10% | 63.0 g |

Procedure

Methylparaben, propylparaben and sodium chloride were dissolved in purified water at 80°C (kettle I). Econazole base, ascorbylpalmitate, lecithin and cholesterol were dissolved in ethanol in a separate kettle at 40°C (kettle II). The ethanol solution was purged with nitrogen during the whole procedure. Both solutions were cooled to room temperature. Kettle I was connected to a high-performance homogenizer (Megatron MT-61; manufacturer: Kenematica, Littau, Lucerne, Switzerland) to effect circulation of the aqueous solution. (Flow rate: 50 liters/minute.) The ethanol solution was injected through a tube from kettle II directly into the homogenizer. (Flow rate: 500 ml/minute: pressure: 0.29 bar (0.02 psi.)) Liposomes having a diameter of less than 2.5 $\mu$m were spontaneously formed and collected in kettle I. After completion of this procedure, hydrochloric acid 10% and perfume were added to kettle I. Hydroxypropylmethyl cellulose was added to effect jellification.

| Technical Data | |
|---|---|
| Homogenizer speed | 8000 rpm |
| Flow rate aqueous solution | 50 l/min. |
| Flow rate ethanol solution | 0.5 l/min. |
| Diameter of the nozzle opening | 6 mm |
| Pressure | $0,1379.10^{-2}$ bar (0.02 psi) |

EXAMPLE IV

| Terconazole (micronized) | 2.0 g |
|---|---|
| Lecithin | 25.0 g |
| Cholesterol | 2.5 g |
| Ethanol | 25.0 g |
| Ascorbyl palmitate | 0.25 g |
| Sodium chloride | 0.2775 g |
| Methylparaben | 0.35 g |
| Propylparaben | 0.025 g |
| Hydrochloric acid 10% | 1.03 g |
| Water purified | 193.5675 g |

Procedure

Methylparaben, propylparaben and sodium chloride were dissolved in water at 80° C in a vessel equipped with a high-performance homogenizer (Polytron PTA 20 SM; manufacturer: Kinematica, Littau-Lucerne, Switzerland). Terconazole, lecithin, ascorbylpalmitate and cholesterol were dissolved in ethanol at 40° C while stirring in a separate vessel. Both solutions were cooled to room temperture. The ethanol solution was then pumped through a tube directly into the aqueous solution; the solutions were simultaneously mixed at high speed. Liposomes having a diameter of less than 2.5 $\mu$m were spontaneously formed and collected in the vessel equipped with the high-performance homogenizer. pH was adjusted to 5.5 by adding hydrochloric acid.

| Technical data | |
|---|---|
| Homogenizer speed | 20,000 rpm |
| Flow rate ethanol solution | 8 ml/min. |
| Diameter of the nozzle opening | 1.0 mm |
| Pressure | $6,895.10^{-2}$ bar (1 psi) |

The liposomes prepared by the present invention were characterized using the following physiochemical procedures.

1. The hydrodynamic properties i.e. mass determination and vesicle radius were determined with an analytical ultracentrifuge (Beckman L-65 with refractive accessories) and by dynamic laser light scattering.

2. Vesicle homogeneity was determined by electronmicroscopy using the freeze fracturing technique.

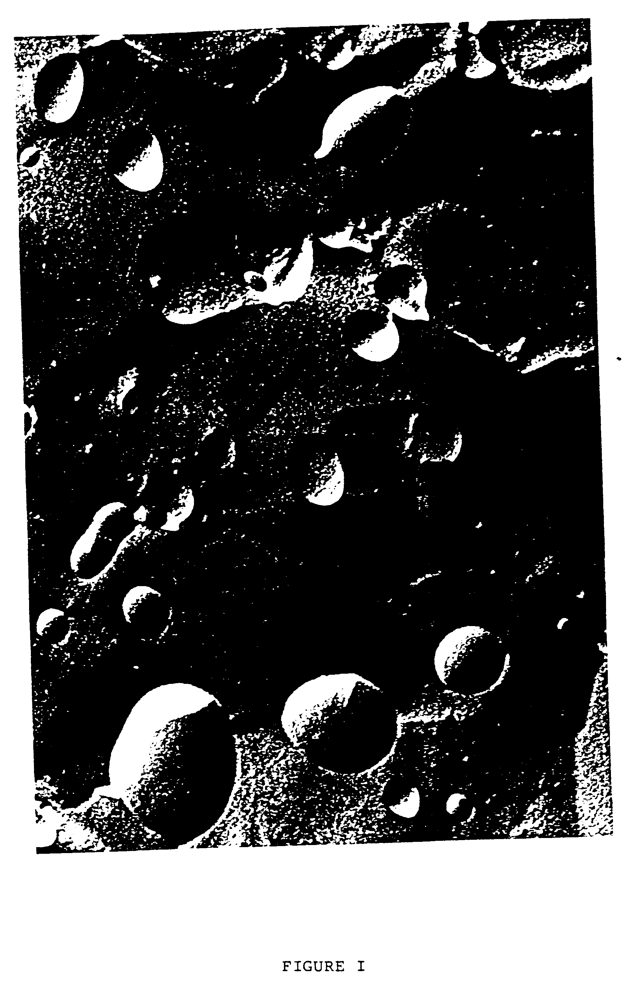

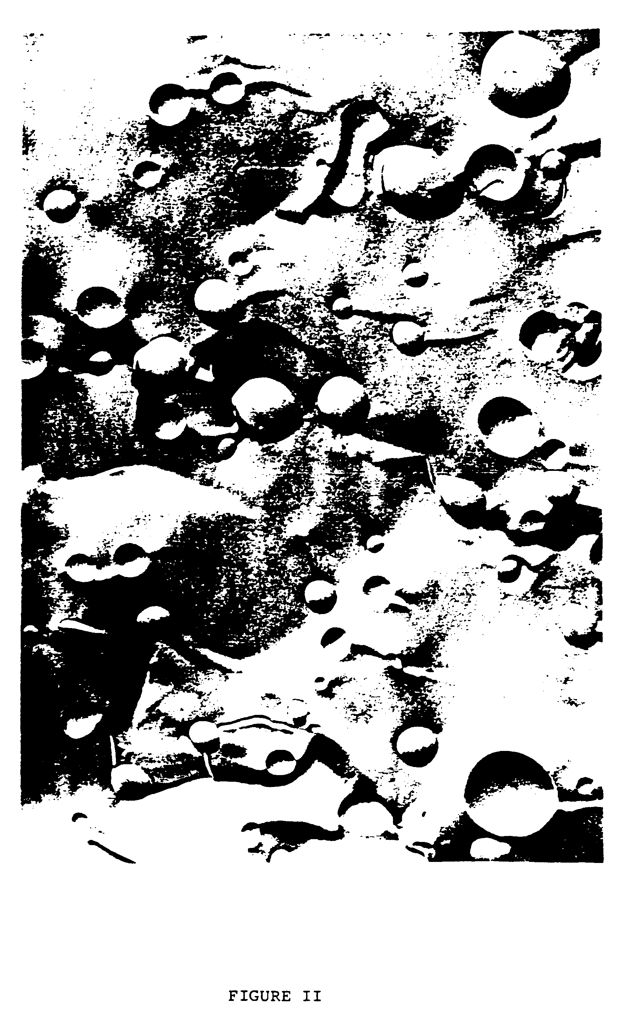

Figure 1 is an electro-micrograph of liposomes prepared according to Example I.

Figure II is an electro-micrograph of liposomes prepared according to Example II.

**Claims**

1. A process for preparing single bilayered liposomes comprising the steps of:

   a) providing a vessel partially filled with an aqueous component;

   b) providing a lipid component dissolved in a suitable organic solvent in a separate vessel;

   c) providing a high speed homogenizer, the mixing means of which are immersed in said aqueous component;

   d) injecting the lipid component, under pressure, through an injecting means directly onto the aqueous component while simultaneously agitating the mixture via the mixing means at high speed to form an aqueous dispersion of lipid, whereupon single bilayered liposomes are formed.

2. A process for preparing single bilayered liposomes comprising the steps of:

   a) providing a vessel partially filled with an aqueous component;

   b) providing an organic component comprised of a lipid component dissolved in a suitable organic solvent in a separate vessel;

   c) providing a high speed homogenizer containing a mixing means;

   d) circulating said aqueous component through said homogenizer;

   e) injecting said lipid component under pressure, through an injecting means directly into said homogenizer;

   f) mixing the solutions at high speed to form an aqueous dispersion of the lipid, and collecting the single bilayered liposomes which form.

3. The process of claim 1 or claim 2, for preparing single bilayered liposomes containing a biologically active material, wherein said lipid component in step (b) further comprises the biologically active material dissolved in the organic solvent.

4. The process of claim 3, wherein a lipophilic biologically active material is present in admixture with the lipid component.

5. The process of claim 4, wherein the lipophilic biologically active material is an antifungal agent.

6. The process of claim 5, wherein the antifungal agent is econazole, terconazole or miconazole.

7. The process of claim 4, wherein the lipophilic biologically active material is a non-steroidal anti-inflammatory agent.

8. The process of claim 4, wherein the lipophilic biologically active material is a prostaglandin.

9. The process of any one of claims 1 to 8 wherein the aqueous component contains an electrolyte.

10. The process of claim 9 wherein the electrolyte is sodium chloride or preferably calcium chloride.

11. The process of any one of claims 1 to 10 wherein the lipid component is a phospholipid.

12. The process of claim 11, wherein the phospholipid is phosphatidylcholine.

13. The process of claim 11 or claim 12 wherein the phospholipid is present in admixture with cholesterol.

14. The process of any one of claims 1 to 13 wherein the organic solvent is ethanol.

15. The process of any one of claims 1 to 14 wherein the mixing means has a speed between 1500 to 20,000 rpm.

16. The process of any one of claims 1 to 15 wherein said injecting means is attached to one or more nozzles.

17. The process of claim 16 wherein the diameter of the nozzle is 0.1 to 10.0 mm.

18. The process of any one of claims 1 to 17 wherein the lipid component is injected into the aqueous component under a pressure of 0.1 - 300 bar.

19. The process of any one of claims 1 to 18 wherein the flow rate of the organic component is 1 to 1000 ml/minute.

20. The process of any one of claims 1 to 19 wherein the aqueous and organic solutions are purged with nitrogen.

21. The process of any one of claims 1 to 20 wherein a suitable thickening agent is added.

22. The process of claim 21 wherein the thickening agent is xanthan gum, hydroxypropyl cellulose or a mixture thereof.

**Revendications**

1. Procédé de préparation de liposomes singuliers à deux couches comprenant les étapes de :
   a) prévoir un récipient partiellement rempli d'un composant aqueux ;
   b) prévoir un composant lipidique dissous dans un solvant organique approprié dans un récipient séparé;
   c) prévoir un homogénéiseur à vitesse rapide, dont les moyens de mélange sont immergés dans ledit composant aqueux ;
   d) injecter le composant lipidique, sous pression, à travers un moyen d'injection, directement sur le composant aqueux tout en agitant simultanément le mélange via les moyens de mélange à vitesse rapide pour former une dispersion aqueuse du lipide pour ainsi former des liposomes singuliers à deux couches.

2. Procédé de préparation de liposomes singuliers à deux couches comprenant les étapes de :
   a) prévoir un récipient partiellement rempli d'un composant aqueux ;
   b) prévoir un composant organique formé d'un composant lipidique dissous dans un solvant organique approprié dans un récipient séparé ;

8

c) prévoir un homogénéiseur à vitesse rapide contenant un moyen de mélange ;

d) faire circuler ledit composant aqueux à travers ledit homogénéiseur ;

e) injecter ledit composant lipidique sous pression, à travers un moyen d'injection, directement dans ledit homogénéiseur ;

f) mélanger les solutions à vitesse rapide pour former une dispersion aqueuse du lipide et recueillir les liposomes singuliers à deux couches qui se forment.

3. Procédé de la revendication 1 ou de la revendication 2, pour la préparation de liposomes singuliers à deux couches contenant une matière biologiquement active, où ledit composant lipidique à l'étape b) contient de plus la matière biologiquement active dissoute dans le solvant organique.

4. Procédé de la revendication 3 où une matière biologiquement active lipophile est présente en mélange avec le composant lipidique.

5. Procédé selon la revendication 4 où la matière biologiquement active lipophile est un agent antifongique.

6. Procédé selon la revendication 5 où l'agent antifongique est éconazole, terconazole ou miconazole.

7. Procédé selon la revendication 4 où la matière lipophile biologiquement active est un agent anti-inflammatoire non stéroïde.

8. Procédé selon la revendication 4, où la matière liphophile biologiquement active est une prostaglandine.

9. Procédé selon l'une quelconque des revendications 1 à 8 où le composant aqueux contient un électrolyte.

10. Procédé selon la revendication 9 où l'électrolyte est le chlorure de sodium ou de préférence le chlorure de calcium.

11. Procédé selon l'une quelconque des revendications 1 à 10 où le composant lipidique est un phospholipide.

12. Procédé selon la revendication 11 où le phospholipide est la phosphatidylcholine.

13. Procédé selon la revendication 11 ou la revendication 12 où le phospholipide est présent en mélange avec du cholestérol.

14. Procédé selon l'une quelconque des revendications 1 à 13 où le solvant organique est l'éthanol.

15. Procédé selon l'une quelconque des revendications 1 à 14 où le moyen de mélange a une vitesse comprise entre 1500 et 20 000 t/mn.

16. Procédé selon l'une quelconque des revendications 1 à 15 où ledit moyen d'injection est attaché à un ou plusieurs injecteurs.

17. Procédé selon la revendication 7 où le diamètre de l'injecteur est de 0,1 à 10,0 mm.

18. Procédé selon l'une quelconque des revendications 1 à 17 où le composant lipidique est injecté dans le composant aqueux sous une pression de 0,1 - 300 bars.

19. Procédé selon l'une quelconque des revendications 1 à 18 où le débit du composant organique est de 1 à 1000 ml/minute.

20. Procédé selon l'une quelconque des revendications 1 à 19 où les solutions aqueuse et organique sont purgées à l'azote.

**21.** Procédé selon l'une quelconque des revendications 1 à 20 où un agent épaississant approprié est ajouté.

**22.** Procédé selon la revendication 21 où l'agent épaississant est la gomme xanthane, l'hydroxypropyl cellulose ou leur mélange.

**Patentansprüche**

**1.** Verfahren zur Herstellung einzelner, doppelschichtiger Liposomen, welches Verfahren die folgenden Stufen umfaßt:
a) Bereitstellen eines teilweise mit einer wässerigen Komponente gefüllten Gefäßes;
b) Bereitstellen einer in einem geeigneten organischen Lösungsmittel aufgelösten Lipidkomponente in einem getrennten Gefäß;
c) Bereitstellen eines Hochgeschwindigkeitshomogenisators, dessen Mischeinrichtungen in die wässerige Komponente eingetaucht sind;
d) Injizieren der Lipidkomponente, unter Druck durch eine Einspritzeinrichtung direkt auf die wässerige Komponente, während das Gemisch gleichzeitig mit Hilfe der Mischeinrichtungen bei hoher Geschwindigkeit gerührt wird, um eine wässerige Dispersion des Lipids zu bilden,wonach einzelne, doppelschichtige Liposomen gebildet werden.

**2.** Verfahren zur Herstellung einzelner, doppelschichtiger Liposomen, welches Verfahren die folgenden Stufen umfaßt:
a) Bereitstellen eines teilweise mit einer wässerigen Komponente gefüllten Gefäßes;
b) Bereitstellen einer organischen Komponente,welche aus einer in einem geeigneten organischen Lösungsmittel aufgelösten Lipidkomponente besteht, in einem getrennten Gefäß;
c) Bereitstellen eines Hochgeschwindigkeitshomogenisators, welcher eine Mischeinrichtung enthält;
d) Zirkulierenlassen der wässerigen Komponente durch den Homogenisator;
e) Injizieren der Lipidkomponente, unter Druck,durch eine Einspritzeinrichtung direkt in den Homogenisator;
f) Vermischen der Lösungen bei hoher Geschwindigkeit zur Bildung einer wässerigen Dispersion des Lipids, und Sammeln der einzelnen, doppelschichtigen Liposomen, welche sich gebildet haben.

**3.** Verfahren nach Anspruch 1 oder 2 zur Herstellung einzelner, doppelschichtiger Liposomen, welche ein biologisch wirksames Material enthalten, wobei die Lipidkomponente in der Stufe (b) zusätzlich das in dem organischen Lösungsmittel aufgelöste, biologisch wirksame Material umfaßt.

**4.** Verfahren nach Anspruch 3, wobei ein lipophiles, biologisch wirksames Material im Gemisch mit der Lipidkomponente vorliegt.

**5.** Verfahren nach Anspruch 4, wobei das lipophile, biologisch wirksame Material ein Antifungusmittel ist.

**6.** Verfahren nach Anspruch 5, wobei das Antifungusmittel Econazol, Terconazol oder Miconezol ist.

**7.** Verfahren nach Anspruch 4, wobei das lipophile, biologisch wirksame Material ein nichtsteroides entzündungshemmendes Mittel ist.

**8.** Verfahren nach Anspruch 4, wobei das lipophile, biologisch wirksame Material ein Prostaglandin ist.

**9.** Verfahren nach einem der Ansprüche 1 bis 8, wobei die wässerige Komponente einen Elektrolyten enthält.

**10.** Verfahren nach Anspruch 9, wobei der Elektrolyt Natriumchlorid oder vorzugsweise Calciumchiorid ist.

**11.** Verfahren nach einem der Ansprüche 1 bis 10, wobei die Lipidkomponente ein Phospholipid ist.

**12.** Verfahren nach Anspruch 11, wobei das Phospholipid Phosphatidylcholin ist.

**13.** Verfahren nach Anspruch 11 oder 12, wobei das Phospholipid im Gemisch mit Cholesterin vorliegt.

**14.** Verfahren nach einem der Ansprüche 1 bis 13, wobei das organische Lösungsmittel Ethanol ist.

**15.** Verfahren nach einem der Ansprüche 1 bis 14, wobei die Mischeinrichtung eine Geschwindigkeit zwischen 1.500 und 20.000 U/min aufweist.

**16.** Verfahren nach einem der Ansprüche 1 bis 15, wobei die Einspritzeinrichtung an einer Düse oder an mehreren Düsen befestigt ist.

**17.** Verfahren nach Anspruch 16, wobei der Durchmesser der Düse 0,1 bis 10,0 mm beträgt.

**18.** Verfahren nach einem der Ansprüche 1 bis 17, wobei die Lipidkomponente unter einem Druck von 0,1 bis 300 bar in die wässerige Komponente eingespritzt wird.

**19.** Verfahren nach einem der Ansprüche 1 bis 18, wobei die Fließgeschwindigkeit der organischen Komponente 1 bis 1.000 ml/min beträgt.

**20.** Verfahren nach einem der Ansprüche 1 bis 19, wobei die wässerige lösung und die organische lösung mit Stickstoff durchgespült werden.

**21.** Verfahren nach einem der Ansprüche 1 bis 20, wobei ein geeignetes Verdickungsmittel zugesetzt wird.

**22.** Verfahren nach Anspruch 21, wobei das Verdickungsmittel Xanthangummi, Hydroxypropylzellulose oder ein Gemisch hievon ist.

FIGURE I

FIGURE II